# EUROPEAN PATENT APPLICATION

(11) **EP 3 750 432 A1**
(43) Date of publication of application: **16.12.2020**
(21) Application number: 19750521.7
(22) Date of filing: 07.02.2019
(51) Int. Cl.: A41D 13/06, A61F 5/00

(54) **DYNAMIC COMPLIMENT FOR PROTECTING AND SUPPORTING THE TIBIAL PLATEAU, KNEECAP AND KNEE**

(30) Priority: 07.02.2018 ES 201800041
(71) Applicant: Fernández Gil, Francisco Javier, 50010 Zaragoza (ES)
(72) Inventor: Fernández Gil, Francisco Javier, 50010 Zaragoza (ES)
(74) Representative: Ungria López, Javier
(86) International application number: PCT/ES2019/000012
(87) International publication number: WO 2019/155099

(57) **Abstract**

The dynamic accessory for protection, bracing, and stabilization of the tibial plateau, kneecap, and knee (1) designed in the specified form (9,20,21,22,25,26), embodied by a single, open, continuous piece, consisting of elastic microfiber fabric, neoprene, foam padding, and breathable and non-breathable fabric that surrounds the perimeter of the user's leg under the knee and in an enveloping maneuver surrounds the tibial plateau and knee, encircling it and closing onto itself and its ends, without covering the popliteal fossa. The stabilizers (3,4,5,31,31'), specially designed (10,15) (11,16) to be situated inside the sectorized pocket (2,12) that is designed in a concave and convex curved shape (17,18,23,24), used to promote the kinesthetic function. They also create an air chamber in order to avoid condensation from sweat and allow ventilation, as well as providing space to adapt to the users leg according to the activity carried out. Connected devices are added to implement the function of the accessory (6,7,8,27,28,28',29,29').

## Description

### THE FIELD OF THE INVENTION

The present invention pertains to the technical field of protecting the knee, applicable to any physical activity in daily life and particularly for athletics.

The invention refers specifically to The Dynamic Accessory for the protection and bracing of the tibial plateau, kneecap, and knee.

### BACKGROUND INFORMATION FOR THE INVENTION

Pull-on knee braces and sleeves of the standard type (made of neoprene, rubber or foam padding, tube-shaped and provided with an opening in the front of the kneecap) present the problem of limited mobility given that they cover the femoral part of the leg above the knee and of the popliteal fossa. As a consequence, they provide less lateral compression and greater discomfort during use. They also depend on the correct colocation or positioning of the knee support.

One option to avoid these problems is the use of lateral stabilizers and femoral and tibial straps, in order to improve the adjustments of the tubular knee braces. However, this system may cause greater discomfort to the user, limit knee mobility or cause kneecap displacement while moving and often requires repositioning; consequently, it entails ineffective bracing.

Another common system to allow knee mobility and precise bracing is the infrapatellar strap and patellar support, however this system does not provide the bracing and lateral stability required by any physical activity in daily life and in particular when practicing sports.

### DESCRIPTION OF THE INVENTION

The present invention is a knee brace that aids and improves the mobility of the knee, bracing and stabilizing the areas corresponding to the tibial plateau, the kneecap and lateral areas of the knee. It uses stabilizers that are inserted into pockets with the given form and the specific bracing systems that have been calibrated according to the physical activity performed.

The dynamic accessory for the protection and bracing of the tibial plateau, kneecap and knee envelops the inferior part of the knee while leaving the popliteal fossa free. It is fashioned from a single piece of elastic microfiber fabric comprised of neoprene, foam padding, and breathable and non-breathable fabric that allows the perfect adaptation to the morphology and anatomy of the perimeter of the inferior, lateral, and superior parts of the kneecap and knee.

The dynamic accessory for the protection and bracing of the tibial plateau, kneecap and knee has a particular and functional design that combines straight and curved lines in all of its contours to best adapt to the morphology and anatomy of every user; it is designed for dynamic movement.

This invention, the dynamic accessory for the protection and bracing of the tibial plateau, kneecap and knee, incorporates at least one pocketed section on its interior face in contact with the user, located above the patellar tendon, the tibial plateau, the lateral ligaments, and around the kneecap. The pocket or pockets are sectioned in order to house the stabilizers that contribute to the bracing and stabilization of the inferior frontal and lateral parts of the knee, and around the kneecap. The pocket or pockets that house the stabilizers have a particular shape that promotes Kinesthetic and neuromuscular functions, and creates an air chamber to avoid the condensation caused by sweat as well as to aid ventilation. The sectioned pocket or pockets are curved at the top. Their sides alternate concave-convex patterns with different radius. The angles in each sectioned pocket help to adapt the dynamic accessory for the protection and bracing of the tibial plateau, kneecap and knee, to the morphology and anatomy of the user. This avoids displacement resulting from functional movements of the knee.

The stabilizers of the dynamic accessory for the protection and bracing of the tibial plateau, kneecap and knee, are made from a single piece of material which optimizes bracing and stabilization of the knee and its lateral ligaments. The central stabilizer is located in the corresponding pocket, to be lined up with the central part of the tibial plateau; it is concave in shape. The lateral stabilizers of the tibial plateau, located in the lateral sections of the sectioned pocket, are lined up with the external and internal parts of the tibial plateau and with the external and internal femoral condyles. They are curved in shape and in the figure of a duck without a bill and oversized head. In another variant of the lateral stabilizers of the tibial plateau, located in the lateral sections of the sectioned pocket, lined up with the external and internal parts of the tibial plateau and with the external and internal femoral condyles, with lateral patellar extension, wrap around the kneecap in curved shape in the figure of a duck without a bill and oversized head; its upper part has extensions in the shape of the edges of the teeth of a saw. The enveloping stabilizers that surround the lateral ligaments are held in the pockets situated in the internal and external laterals of the knee, and are in the shape of interlocked flat chain links.

In another variant the lateral stabilizers located in the internal and external sides of the knee, housed in the sectioned pockets of the internal and external side of the knee, present an elongated oval shape. The lateral stabilizers situated in the internal and external sides of the knee provide more stability to the patella with no need of a larger contact surface. This provides a greater feeling of freedom while moving, according to the physical activity carried out.

The dynamic accessory for the protection and bracing of the tibial plateau, kneecap and knee, made from a single piece of elastic microfiber fabric, neoprene, foam padding, and breathable and non-breathable fabric, is fastened by a hook-and-loop system on interior and exterior face of the material, so it sticks together through sewing, thermal-sealing, or whatever system of fixation, that aids in the perfect closing of the knee brace to fit the morphology and anatomy of the user.

In another implementation, the dynamic accessory for the protection and bracing of the tibial plateau, kneecap and knee made from a single piece of elastic microfiber fabric, neoprene, foam padding, and breathable and non-breathable fabric, increases the height of the front central section, which corresponds to the sectioned pocket and its sides, to protect a greater surface area of the patella, and increases the height of the posterior lateral part of the dynamic accessory for the protection and bracing of the tibial plateau, kneecap and knee, in an enveloping maneuver over the kneecap and the patella to envelope a greater surface area of the patella, increasing knee stability, according to the physical activity carried out.

Another possible implementation of the dynamic accessory for the protection and bracing of the tibial plateau, kneecap and knee, subject of the invention, incorporates two elastic tapes either with and/or without tension, joined together (by gluing, sewing, thermo-sealing or any fixing system) to the internal or external central anterior part of the dynamic accessory for the protection and bracing of the tibial plateau, kneecap and knee which cross at the level of the anterior cruciate ligament, and extend lengthwise, towards the lateral ligaments. In this way, greater stability is achieved in the patella, stabilizing the anterior and lateral ligaments, during the rotations made, depending on the physical activity carried out.

One other possible implementation of the dynamic accessory for the protection and bracing of the tibial plateau, kneecap and knee, subject of the invention, incorporates two elastic tapes either with and/or without tension, joined (by gluing, sewing, thermo-sealing or any fixing system) to the internal or external central posterior part of the dynamic accessory for the protection and bracing of the tibial plateau, kneecap and knee that cross at the level of the hind cruciate ligament, and extend lengthwise, towards the collateral ligaments. In this way, greater stability is achieved in the hind cruciate ligament and collateral ligaments, preserving the feeling of freedom of the popliteal hollow, during the physical activity carried out.

Another different implementation of the dynamic accessory for the protection and bracing of the tibial plateau, kneecap and knee, also uses the system of hook and loop system to fix the aid. It has at least one sectioned pocket that houses the central stabilizer for the tibial plateau, and for the lateral stabilizers of the tibial plateau and kneecap. In this version it incorporates a longitudinal elastic strap both with and/or without tension in the middle part of the exterior face, creating a cohesive method of easy but secure closing by sewing, heat-sealing, or whatever method of fastening is chosen.

In another form of implementation, the longitudinal elastic strap works together with a transverse elastic band, both with and/or without tension, joined together above the posterior lateral part of the external face of the dynamic accessory for the protection and bracing of the tibial plateau, kneecap and knee, that coincides with the hind lateral external part of the leg, which stays fix through, sewing, heat-sealing, or whatever method of closing chosen. In this way the knee is more stable and braced, according to the physical activity and the needs of the user.

In one more variant of the previous rendering, the dynamic accessory for the protection and bracing of the tibial plateau, kneecap and knee, object of this invention, incorporates a bracing strap with one or two buckles on one of its ends, and the closing system of hooks and loops on the opposite end. The bracing strap superimposes over the longitudinal elastic strap, and is fastened to one of its ends, (under the transversal elastic strap both with and/or without tension), to the posterior lateral external of the dynamic accessory for the protection and bracing of the tibial plateau, kneecap and knee, that coincides with the hind part of the lateral external of the leg (through heat sealing, sewing, heat-seal, or whatever method of closing chosen). Once the dynamic accessory for the protection and bracing of the tibial plateau, kneecap and knee is placed and braced above the perimeter of the users leg on the inferior and lateral part of the kneecap and knee, the bracing strap encircles the dynamic accessory for the protection and bracing of the tibial plateau, kneecap and knee at the level of the longitudinal elastic strap. In this way it achieves more stability, lessening impacts and vibrations above the patellar tendon and decreasing pain through pressure as well as bracing the knee during physical activity.

In a further variation of the dynamic accessory for the protection and bracing of the tibial plateau, kneecap and knee made in a single piece, it incorporates a bracing tongue attached through sewing, heat-seal, or whatever method of closing at one of its ends, to the posterior lateral external part of the dynamic accessory for the protection and bracing of the tibial plateau, kneecap and knee, that coincides with the posterior lateral external part of the leg. Once the dynamic accessory for the protection and bracing of the tibial plateau, kneecap and knee is placed around the perimeter of the users leg at the inferior lateral sides of the kneecap and knee, it positions and braces the area. The other end of the bracing tongue is joined to the dynamic accessory through the closing system of hooks and loops from which the bracing tongue on the interior face becomes the end of the dynamic accessory, protecting and bracing of the tibial plateau, kneecap and knee on its exterior face. By fixing the free end of the bracing tongue above the Dynamic accessory for protection and bracing, at the level of the tibial zone, greater stability is obtained as well as a decrease in the impacts and vibrations above the patellar tendon. Any pain is reduced by means of pressure and bracing of the knee according the physical activity performed.

Another variation of the dynamic accessory for the protection and bracing of the tibial plateau, kneecap and knee made in a single piece, from materials of elastic microfiber fabric comprised of neoprene, foam padding, and breathable and non-breathable fabric, shows the dynamic complement closed upon itself on both of its ends (through sewing, heat-seal, or whichever method of closing chosen), surrounding the perimeter of the users leg under the knee in an enveloping maneuver over the kneecap and the patella with no need for the closing system of hooks and loops.

The variation described in the previous paragraph of the dynamic accessory for the protection and bracing of the tibial plateau, kneecap and knee in turn allows one further variation consisting of an increase in the height of the front central section, which corresponds to the sectioned pocket and its sides, to protect and cover a greater surface area of the patella, improving knee stability.

In another implementation of the dynamic accessory for the protection and bracing of the tibial plateau, kneecap and knee (made in one continuous piece, from materials of elastic microfiber fabric comprised of neoprene, foam padding, and breathable and non-breathable fabric), is comprised of three sections that constitute the entirety when together : a central part on the interior face, which is in contact with the user, located in a sectioned pocket that has a curved shape on its upper and lower side and has concave and convex curves on each side. This pocket houses the central stabilizer of the tibial plateau and the lateral stabilizers of the tibial plateau and kneecap; two further lateral sections, one on each side of the exterior face of the central section, that together with the central section constitute the complete system. The central section that corresponds to the sectioned pocket has a strategically situated slit on one of its ends, through which the opposite lateral section of the dynamic accessory for the protection and bracing of the tibial plateau is introduced. This allows it to surround the knee, leg and kneecap below the knee completely by closing on itself. The form of this implementation of the dynamic accessory for the protection and bracing of the tibial plateau, kneecap and knee made from one continuous piece, also alleviates impacts and vibrations above the patellar tendon and reduces any pain, adapting to the morphology and anatomy of the knee and leg of the user, in a way that reinforces the bracing of the inferior side of the kneecap and of the knee. In this way we achieve greater stability of the knee according to the physical activity performed. This specific layout is applicable when the need to brace is higher.

The embodied form described in the previous paragraph of the dynamic accessory for the protection and bracing of the tibial plateau, kneecap and knee presents in turn, the variants of implementation described previously and are consistent with:
a) Additional positioning, through the closing system by hooks and loops on the exterior face of the central section.
b) Increasing the height of the front central section, which corresponds to the sectioned pocket and its sides, to cover a greater surface area of the patella and increases the height of the posterior lateral part of The dynamic accessory for the protection and bracing of the tibial plateau, kneecap and knee, in an enveloping maneuver over the kneecap and the patella to cover a greater surface area of the patella, increasing knee stability.
c) Incorporating two elastic tapes (with and/or without tension), joined together (by gluing, sewing, heat-sealing or any fixing system), to the internal or external central front part of the dynamic accessory for the protection and bracing of the tibial plateau, kneecap and knee, which cross at the level of the anterior cruciate ligament, and extend longitudinally, towards the lateral ligaments.
d) Incorporating two elastic tapes (with and/or without tension), joined together (by gluing, sewing, heat-sealing or any fixing system), to the external or internal central back part of the dynamic accessory for the protection and bracing of the tibial plateau, kneecap and knee , which cross at the level of the posterior cruciate ligament, and extend longitudinally, towards the collateral ligaments.
e) Incorporating two stabilizers of the internal and external lateral faces of the knee, housed in the pockets located in the external and internal lateral part of the knee, in an elongated oval shape.

Another embodied form of the dynamic accessory for the protection and bracing of the tibial plateau, kneecap and knee (made in one continuous piece of elastic microfiber fabric, neoprene, foam padding, and breathable and non-breathable fabric), comprised of one section encircling the low frontal part of the knee, where at least one sectioned pocket is located in the inner part. This pocket houses the central stabilizer of the tibial plateau, the two lateral stabilizers of the tibial plateau and kneecap, as well as the sectioned pockets that house the two pairs of stabilizers surrounding the two lateral ligaments in the form of a flat chain. The sectioned pocket or pockets are curved at the top and alternating concave-convex curves on the side with different radius and angles on each side of the sectioned pockets. The dynamic accessory for the protection and bracing of the tibial plateau, kneecap and knee, made from a single continuous piece has, in this embodied form, two subsections of different length, ascending towards the users thigh, wrapping around the anterior part of the kneecap and knee, crossing over the upper edge, leaving the popliteal fossa free. On its interior face, and in contact with the skin, there is a prolongation of the sectioned pocket or pockets, that houses two pairs of stabilizers that wrap around the lateral ligaments in the form of a flat chain, as well as the extension of the lateral stabilizers of the tibial plateau, in the form of the teeth on the edge of the saw, that wrap around the kneecap, adapting to the morphology and anatomy of the knee providing greater support, stability and comfort.

The embodied form described in the previous paragraph of the dynamic accessory for the protection and bracing of the tibial plateau, kneecap and knee presents the variants of implementation described previously and are consistent with:
a) Incorporating an elastic longitudinal strap (with and/or without tension) in the central part of the exterior face of one continuous piece fixed by sewing, heat-sealing or attached by the chosen system of fixation.
b) Incorporating an elastic longitudinal strap that is complemented with another elastic transversal strap,(both of them with and/or without tension), of one continuous piece above the exterior posterior lateral of the dynamic accessory for the protection and support of the tibial plateau, kneecap and knee that coincides with the external posterior lateral of the leg, through gluing, sewing, heat-seal or whichever system of fixation chosen.
c) Incorporating two elastic tapes (with and/or without tension), joined together (by gluing, stitching, heat-sealing or any fixing system of choice), to the internal or external central front part of the dynamic accessory for the protection and bracing of the tibial plateau, kneecap and knee, which cross at the level of the anterior cruciate ligament, and extend longitudinally, towards the lateral ligaments.
d) Incorporating two elastic tapes (with and/or without tension), joined together (by gluing, sewing, heat-sealing or any fixing system), to the external or internal central back part of the dynamic accessory for the protection and bracing of the tibial plateau, kneecap and knee, which cross at the level of the posterior cruciate ligament, and extend longitudinally, towards the collateral ligaments.
e) Incorporating a bracing strap with one or two buckles on one of its ends (and a closing system of hook and loop on the opposite end to close upon itself), that superimposes above the elastic longitudinal strap, bracing the kneecap and knee under the elastic transversal strap with one of the ends to the external posterior lateral of the dynamic accessory for the protection and bracing of the tibial plateau, coinciding with the external posterior lateral of the leg, through gluing, sewing, heat-seal or whichever system of fixation chosen.
f) Incorporating a bracing tongue (attached through sewing, heat-seal or any other system of fixation), from one of the ends to the external posterior lateral of the dynamic accessory for the protection and bracing of the tibial plateau, kneecap and knee, that coincides with the external posterior lateral of the leg. Once the dynamic accessory for the protection and bracing of the tibial plateau, kneecap and knee is both placed and braced around the perimeter of the user's leg at the inferior and lateral parts of the knee and kneecap, the bracing tongue is fastened to the dynamic accessory for protection and bracing through the hook and loop closing system provided by the bracing tongue on its interior side, just as the Dynamic accessory for protection and bracing does on its exterior side.
g) The embodied form whose section covers the inferior and frontal part of the knee comprises three further sections; a central section with and interior face in contact with the skin of the user, placed at least at the level of the sectioned pocket, and two lateral sections, one on each side of the central section; the central section, which corresponds with the sectioned pockets, has a strategically located slit on one of its ends, through which the opposite lateral section of the dynamic accessory is introduced for the protection and bracing of the tibial plateau, kneecap and knee that allows it to perimetrically surround the leg of the user under the knee.

### BRIEF DESCRIPTION OF THE DRAWINGS

The attached drawings show possible forms of creating the knee brace, constituted with the invention and provided as a non-limiting example.

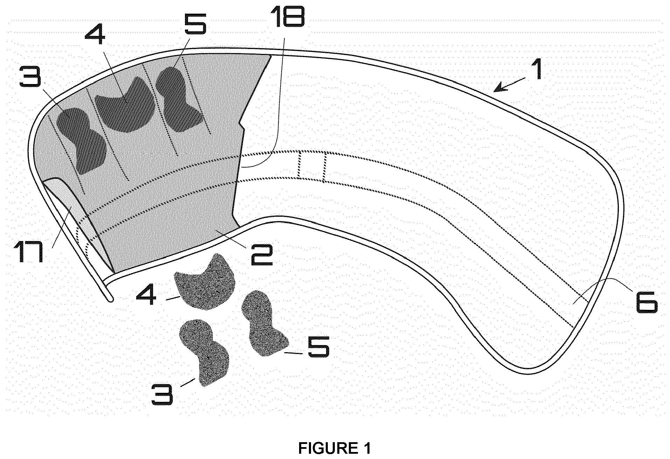

Figure 1 shows the dynamic accessory for the protection and bracing of the tibial plateau, kneecap and knee (1) (made in one continuous piece, of elastic microfiber fabric comprised of neoprene, foam padding, and breathable and non-breathable fabric), in one of its forms, with details of the sectioned pockets (2) and their sides (17, 18) with the shape of alternating concave-convex curves with varying radius and angles on each side of the sectioned pockets; the sectioned pocket houses the central stabilizer for the tibial plateau (4), and the lateral stabilizers of the tibial plateau and kneecap (3,5), of the given configuration, detailed by the stabilizers (3,4,5); and the longitudinal elastic strap (6)(with and/or without tension), attached to the dynamic accessory for the protection and bracing of the tibial plateau, kneecap and knee (1) on its exterior face.

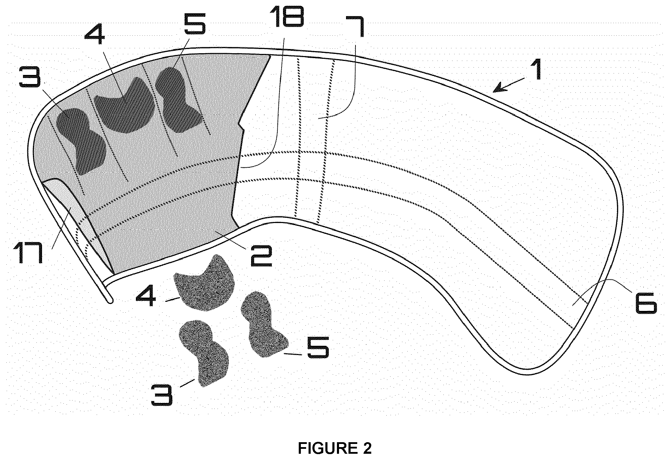

Figure 2 corresponds to a variation of implementation of the dynamic accessory for the protection and bracing of the tibial plateau, kneecap and knee (1) (made in one continuous piece of elastic microfiber fabric, neoprene, foam padding, and breathable and non-breathable fabric), in one of its conformations, with details of the sectioned pocket (2) and the laterals (17,18) with the form of alternating concave-convex curves with different radius and angles on each side of the sectioned pocket. The sectioned pocket houses the central stabilizer of the tibial plateau (4) and the lateral stabilizers of the tibial plateau and kneecap (3,5), of a particular shape, detailed by the stabilizers (3,4,5); and the elastic longitudinal strap (6) and the elastic transversal strap (7), both (with and/or without tension), conjoined with the dynamic accessory for the protection and bracing of the tibial plateau, kneecap and knee (1) on the exterior face.

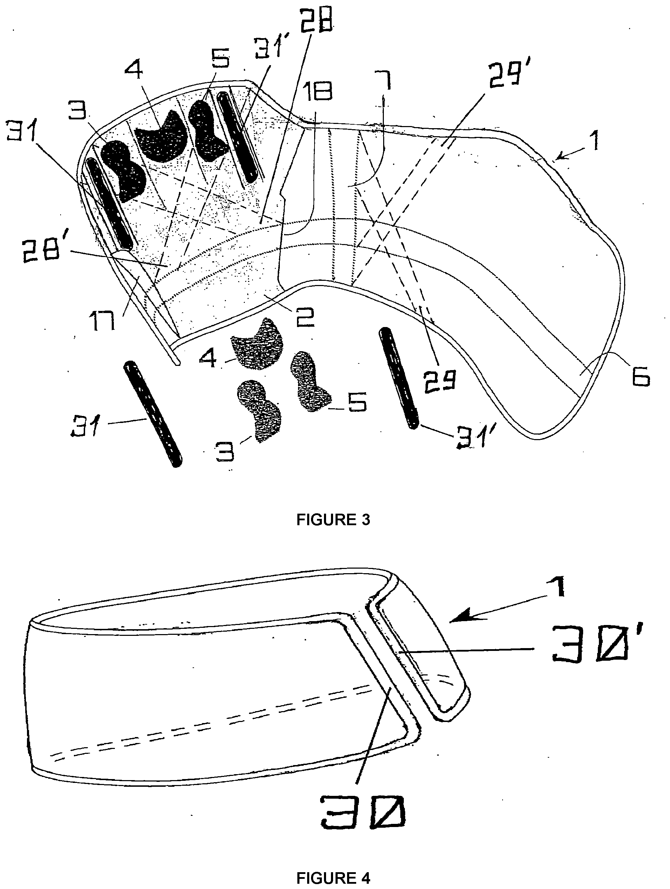

Figure 3 is a view of the alternative implementation shown in (Figure 2), where the height of the front central section increases, which corresponds to the sectioned pockets (2) and its sides (17 y 18), to protect a greater surface area of the patella. The height of the posterior lateral part of the dynamic accessory for the protection and bracing of the tibial plateau, kneecap and knee (1) increases its height and in an enveloping maneuver, closes on a greater patella surface, ensuring knee stability. In addition to the longitudinal elastic strap (6) and the transverse elastic strap (7), both with and/or without tension, together with the dynamic complement of protection and support of the tibial plateau, patella and knee (1), incorporates two elastic tapes (29,29') (with and/or without tension), joined together (by gluing, sewing, thermo-sealing or any fixing system), to the external or internal central back part of The dynamic accessory for the protection and bracing of the tibial plateau, kneecap and knee (1), which cross at the level of the posterior cruciate ligament, and extend longitudinally, towards the collateral ligaments, as well as two elastic bands (28,28') (with and/or without tension), joined together (by gluing , stitching, heat-sealing or any fixation system chosen), to the internal or external central front part of the dynamic accessory for the protection and bracing of the tibial plateau, kneecap and knee (1), which cross at the level of the anterior cruciate ligament, and extend lengthwise, towards the lateral ligaments. Finally, in the sectorized pocket (2), two stabilizers (31,31 ') of the internal and external lateral faces of the knee are incorporated, housed in the pockets located in the external and internal lateral area of the knee, which are shaped as an elongated oval.
Figure 4 is an alternate implementation of the dynamic accessory for the protection and bracing of the tibial plateau, kneecap and knee(1), (made in a single piece based on elastic microfiber, neoprene, foams and breathable and non-breathable textiles), where the dynamic complement is shown, closing on itself, through the joint connection between its two lateral ends (30,30'), (by gluing, sewing, thermo-sealing or any other fixation system), surrounding the perimeter of the user's leg below the knee, and in an enveloping maneuver over the kneecap and knee, without the need to use hook and loop closing system.

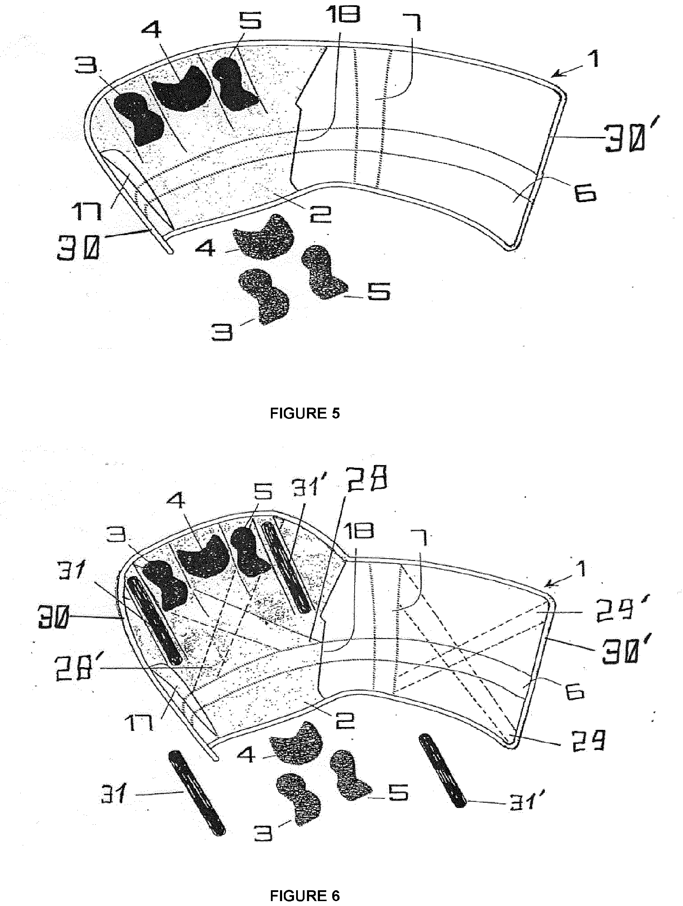

Figure 5 corresponds to a variant of implementation of the dynamic accessory for the protection and bracing of the tibial plateau, kneecap and knee (1) and an alternate implementation shown in (Figure 4), (made in one continuous piece and made of elastic microfiber fabric, neoprene, foam padding, and breathable and non-breathable fabric), in one of its conformations, with details of the sectioned pocket (2) and the laterals (17,18) with the form of alternating concave-convex curves with different radius and angles on each side of the sectioned pocket; the sectioned pocket houses the central stabilizer of the tibial plateau (4) and the lateral stabilizers of the tibial plateau and kneecap (3,5), of a particular form, detailed by the stabilizers (3,4,5); and the elastic longitudinal strap (6) as well as the elastic transversal strap (7), (both of them with and/or without tension), conjoined with The dynamic accessory for the protection and bracing of the tibial plateau, kneecap and knee (1). The dynamic complement of protection and support of the tibial plateau, kneecap and knee (1) joins its two lateral ends (30,30') (by gluing, sewing, heat-sealing or any fixing system), surrounding the user's leg perimetrically below the knee, and in an enveloping maneuver over the kneecap and knee, without the need to use hook and loop closure systems.
Figure 6 is a view of the alternative implementation (shown in Figure 5), where an increase in height is displayed in its front central section, which corresponds to the sectorized pockets (2) and its sides (17,18) to cover a greater surface area of the patella, implementing knee stability. In addition to the elastic longitudinal strap (6) and the elastic transversal strap (7), (both of them with and/or without tension, conjoined with the dynamic accessory for the protection and bracing of the tibial plateau, kneecap and knee) (1) incorporates two elastic tapes (29,29') (with and/or without tension, joined together by gluing, sewing, thermo-sealing or any fixing system), to the external or internal central back part of the dynamic complement of protection and securing the tibial plateau , patella and knee (1), which cross at the level of the posterior cruciate ligament, and which extend lengthwise towards the collateral ligaments, as well as two elastic bands (28,28') (with and/or without tension), joined together (by gluing, sewing , heat-sealed or any other fixation system), to the internal or external central front part of the dynamic complement of protection and support of the tibial plateau, patella and knee (1), which cross at the level of the anterior cruciate ligament, and extend lengthwise, towards the lateral ligaments. Finally, in the sectorized pocket (2) two stabilizers (31,31') of the internal and external lateral faces of the knee are incorporated, housed in the pockets located in the external and internal lateral area of the knee, which are shaped as an elongated oval.

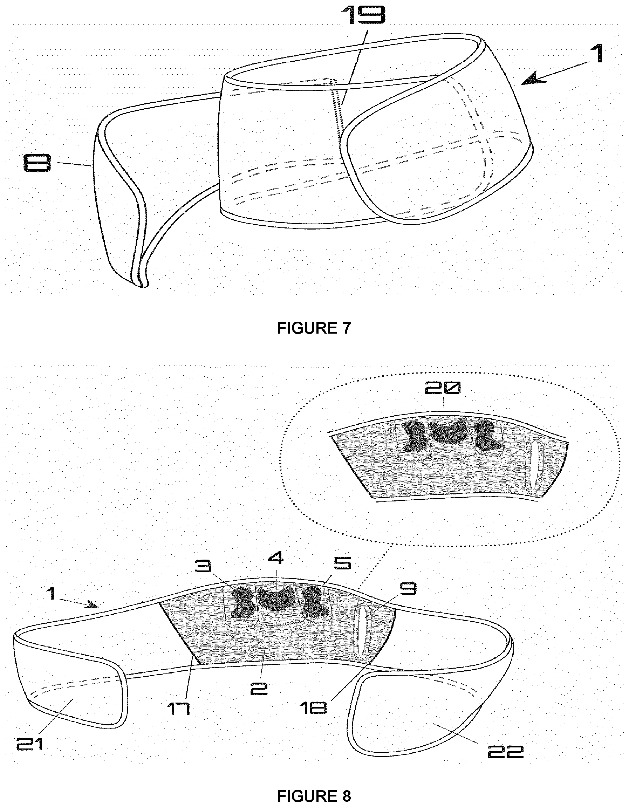

Figure 7 is an alternate implementation of the dynamic accessory for the protection and bracing of the tibial plateau, kneecap and knee (1) (made in one continuous piece of elastic microfiber, neoprene, foam padding, and breathable and non-breathable fabric), fitted with a bracing tongue (8) attached by one of its ends (19) to the external posterior lateral part of The dynamic accessory for the protection and bracing of the tibial plateau, kneecap and knee (1).
Figure 8 shows a view of the alternate implementation of the dynamic accessory for the protection and bracing of the tibial plateau, kneecap and knee (1) (made in one continuous piece of elastic microfiber fabric, neoprene, foam padding , and breathable and non-breathable fabric), that has three sections: a central (20) of which details are presented overexposed, inside the circle; and two lateral sections (21,22), one on each side of the central section (20). Details of the sectioned pocket (2) and its laterals (17,18) with the form of alternating concave-convex curves on each side. The sectioned pocket (2) houses the central stabilizer of the tibial plateau (4), and the lateral stabilizers of the tibial plateau and kneecap (3,5) of a particular form. The central section (20), corresponds with the sectioned pocket (2) has a (9) strategically situated slit on one of its ends, through which the opposite end is introduced, which in this case (Figure 8) corresponds to the side section (21) of The dynamic accessory for the protection and support of the tibial plateau, kneecap and knee (1).

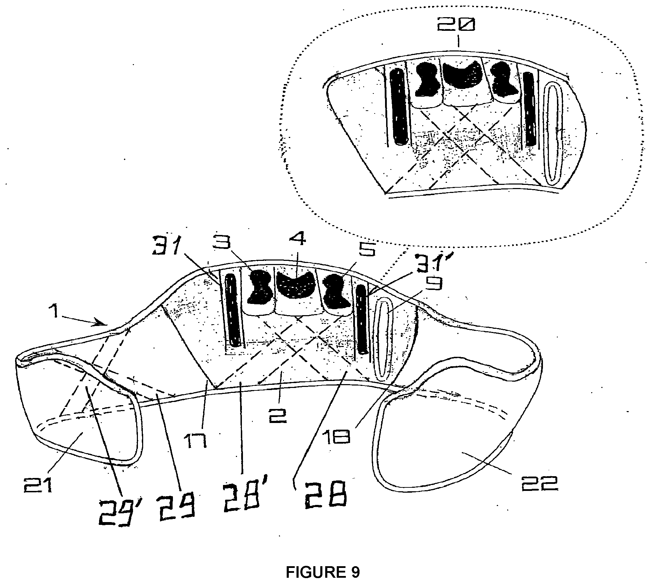

Figure 9 is an alternate implementation shown in (Figure 8), where an increase in height is shown, in its front central section (20), to cover a greater surface area of the patella, as well as, an increase in the height of the posterior lateral part of the two lateral sections (21,22) to, in an enveloping maneuver, close on a larger patella surface, resulting in better knee stability. It incorporates two elastic tapes (28,28')(with and/or without tension), joined together (by gluing, sewing, thermo-sealing or any fixing system), to the internal or external central front part of the dynamic accessory for the protection and bracing of the tibial plateau, kneecap and knee (1), which cross at the level of the anterior cruciate ligament, and extend longitudinally, towards the lateral ligaments, as well as two elastic tapes (29,29')(with and/or without tension), joined together (by gluing, stitching, heat-sealing or any fixing system), to the external or internal central back part of The dynamic complement of protection and fastening of the tibial plateau , patella and knee (1), which cross at the level of the posterior cruciate ligament, and extend lengthwise towards the collateral ligaments. Finally, in the sectorized pocket (2) two stabilizers (31,31 ') of the internal and external lateral faces of the knee are incorporated, housed in the pockets located in the external and internal lateral zone of the knee, and which are shaped as an elongated oval.

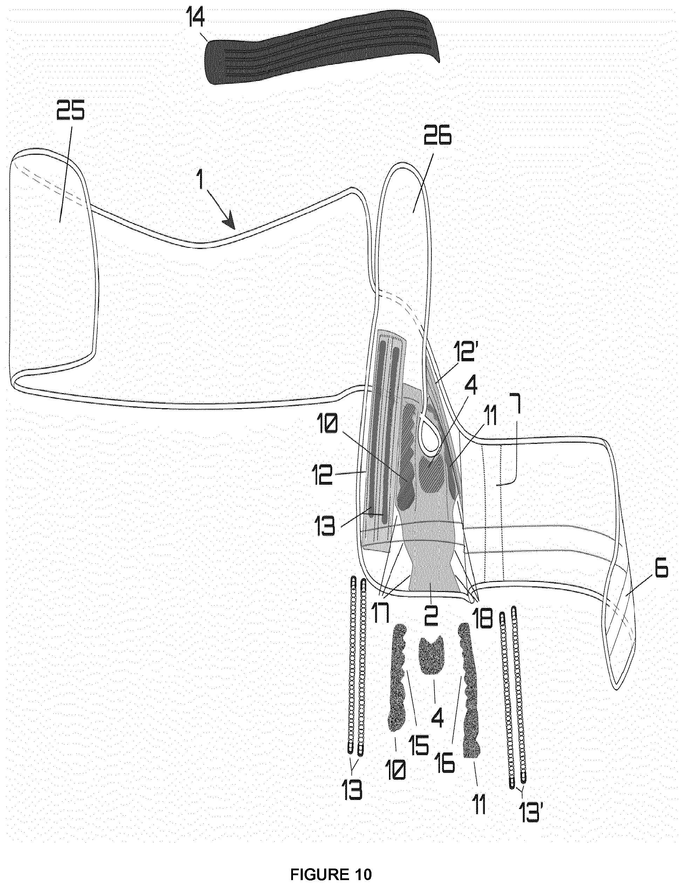

Figure 10 shows a view of an alternate implementation of the dynamic accessory for the protection and bracing of the tibial plateau, kneecap and knee (1) (made in one continuous piece of elastic microfiber fabric, neoprene, foam padding, and breathable and non-breathable fabric), with details of the central stabilizer of the tibial plateau (4), of the lateral stabilizers of the tibial plateau and kneecap (10,11) with its extensions in the shape of the teeth at the edge of a saw (15,16); details of the stabilizers enveloping the lateral ligaments with the shape of a flat chain (13,13'); details of the elastic longitudinal strap (6) and transversal strap (7), (both of them with and/or without tension) attached to the dynamic accessory for the protection and support of the tibial plateau, kneecap and knee (1) on its exterior face; details of the two subsections of different length (25,26), that wrap around the kneecap and knee; and details of the support strap (14) to the thigh of the user, from the dynamic accessory for the protection and support of the tibial plateau, kneecap and knee (1), through a hook and loop closing method. Details of the pocket (2) and its laterals (17,18) in the shape of alternating concave-convex curves with different radius and angles on each side and details of the pockets (12,12') that house the stabilizers (13,13').

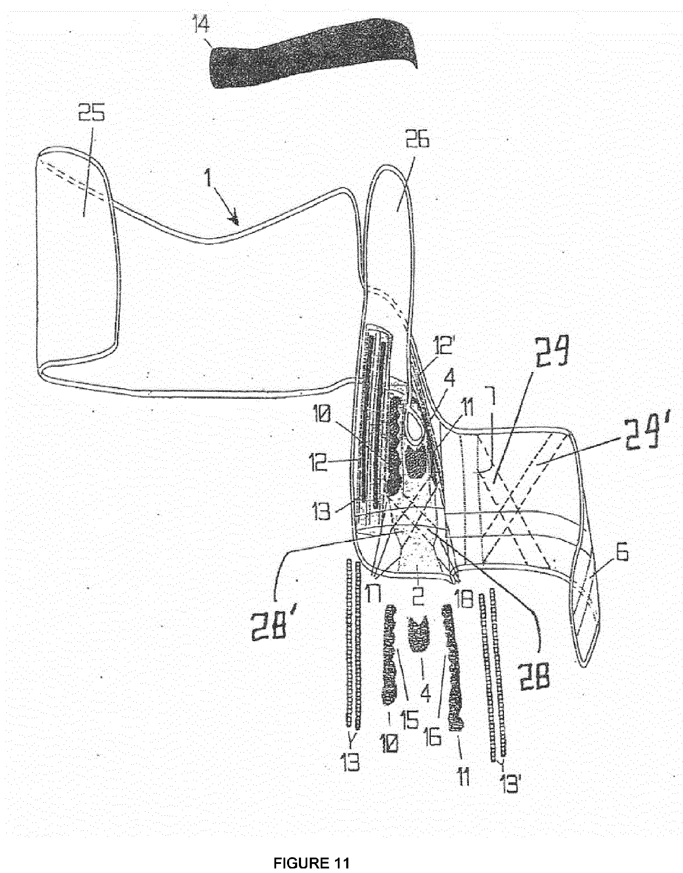

Figure 11 is an alternate implementation shown in (Figure 10), which incorporates two elastic tapes (29,29') (with and/or without tension), held together (by gluing, sewing, thermo-sealing or any fixing system), to the external or internal central back part of the dynamic accessory for the protection and bracing of the tibial plateau, kneecap and knee (1), which cross at the level of the posterior cruciate ligament, and which extend longitudinally towards the collateral ligaments, as well as two elastic bands (28,28') (with and/or without tension), held together (by gluing, stitching, heat-sealing or any fixing system), to the internal or external central front part of the dynamic complement for protection and support of the tibial plateau, patella and knee (1), which cross the height of the anterior cruciate ligament, and extend longitudinally, towards the lateral ligaments.

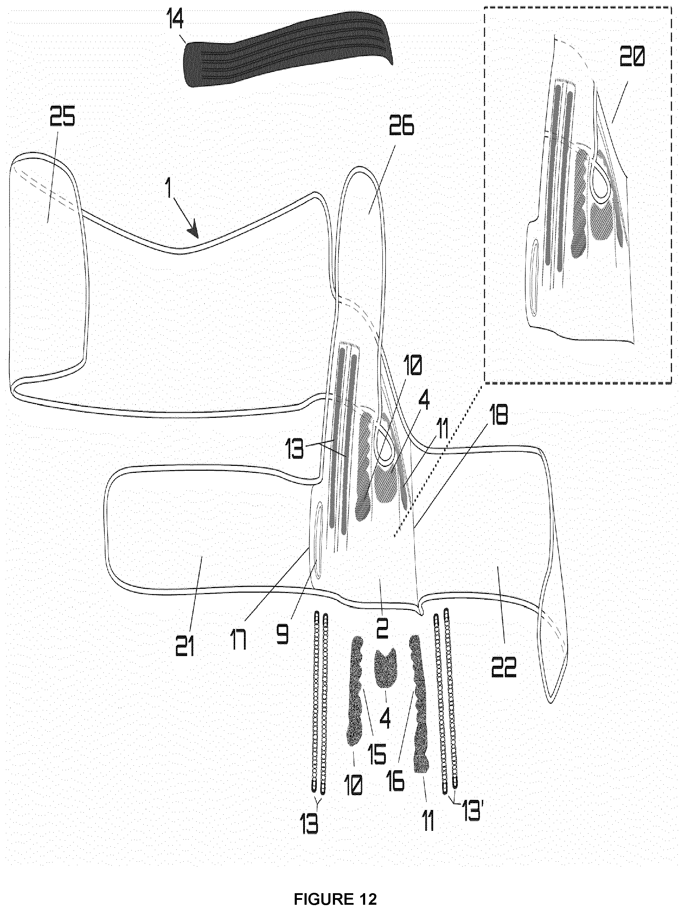

Figure 12 is a view of the alternate implementation shown in (Figure 10). At the base it shows three sections: a central (20) which is presented over-exposed, inside of a square, and two lateral sections (21,22) one on each side of the central section (20). Details of the sectioned pocket (2) and its laterals (17,18) in the shape of concave and convex curves and different radius and angles on each side. Details of the sectioned pocket (2) that houses the central stabilizer of the tibial plateau (4); of the lateral stabilizers of the tibial plateau and kneecap (10,11) with its extensions in the shape of the teeth on the edge of a saw (15,16); and of the stabilizers surrounding the lateral ligaments, in the shape of a flat chain (13,13'). Details of the two subsections of different length (25,26) that wrap around the knee and kneecap; and details of the support strap (14) to the thigh of the user. The central section (20), that corresponds to the sectioned pocket (2) has a (9) strategically situated slit on one of its ends, through which the opposite end is introduced, which in this case (Figure 12) corresponds to the side section (22) of the dynamic accessory for the protection and support of the tibial plateau, kneecap and knee (1).

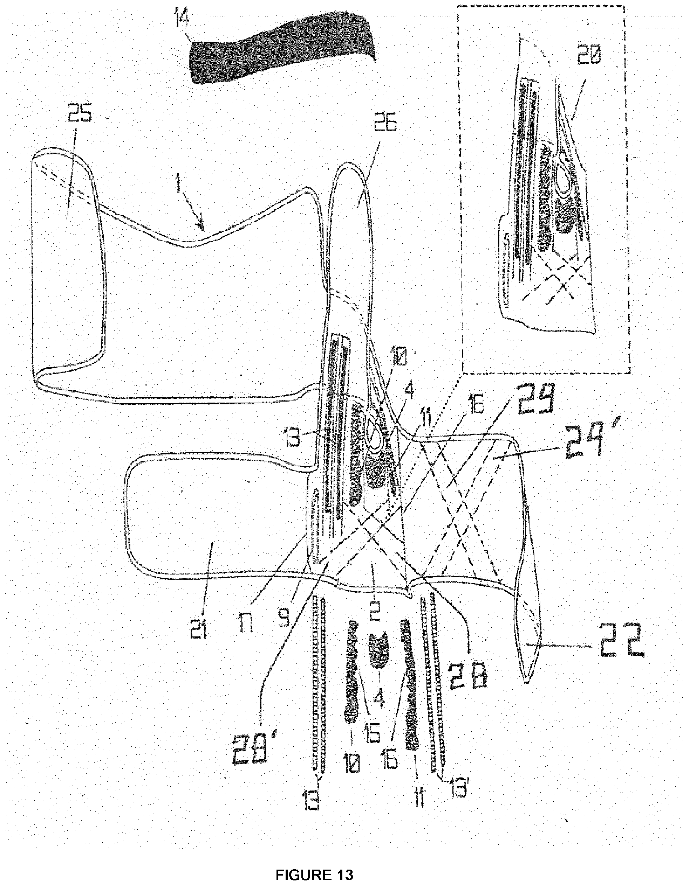

Figure 13 is an alternate implementation shown in (Figure 12), which incorporates two elastic tapes (29,29') (with and/or without tension), joined together (by gluing, sewing, thermo-sealing or any other fixing system), to the external or internal central back part of the dynamic accessory for the protection and bracing of the tibial plateau, kneecap and knee (1), which cross at the level of the posterior cruciate ligament, and which extend longitudinally towards the collateral ligaments, as well as two elastic tapes (28,28') (with and/or without tension), joined together (by gluing, stitching, heat-sealing or any fixing system), to the internal or external central front part of the dynamic accessory for the protection and bracing of the tibial plateau, kneecap and knee (1), which cross at the level of the anterior cruciate ligament, and extend longitudinally, towards the lateral ligaments. Detail of the sectorized pocket (2), where the two elastic tapes (28,28') are added.

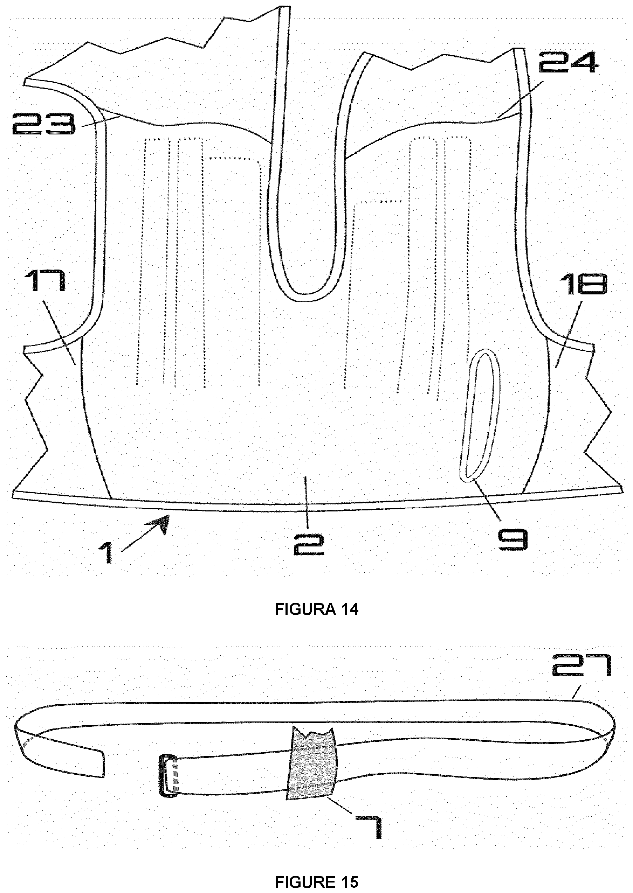

Figure 14 is a partial view of the inner face of the central section of the dynamic accessory for the protection and bracing of the tibial plateau, kneecap and knee (1) and specifically of the sectorized pocket (2). There is a slit (9) on one of its sides, the upper ends (23 and 24) with a sinuous shape and the sides (17,18) with the shape of a concave and convex curve with different radius and angles on each side.
Figure 15 is an alternate implementation of the dynamic accessory for the protection and bracing of the tibial plateau, kneecap and knee (1), which shows the fastening strap with a buckle at one end and hook and loop closure system at the opposite end. Detail of the elastic cross strap (7), which holds the bracing strap (27) at one end.

### DETAILED DESCRIPTION OF ONE POSSIBLE EMBODIMENT

Figure 11 represents a preferred embodiment of the dynamic accessory for the protection and bracing of the tibial plateau, kneecap and knee (1) (built in one continuous piece and made of elastic microfiber fabric, neoprene, foam padding, and breathable and non-breathable fabric), encircling the inferior part of the knee while leaving the popliteal fossa free, that shows on the interior face the sectioned pocket (2), which respectively houses the central stabilizer of the tibial plateau (4), and the lateral stabilizers of the tibial plateau and kneecap (10,11) in the shape of a curve , like a silhouette of a duck without a bill and oversized head, and with extensions in the form of the teeth on the edge of a saw in its superior part (15,16); the sectioned pockets (12, 12') that house two pairs of enveloping stabilizers of the two lateral ligaments (13,13') with the shape of a flat chain. It incorporates a longitudinal elastic strap in the middle part of its exterior part (6) and in the posterior lateral part of the dynamic accessory for the protection and support of the tibial plateau, kneecap and knee (1), an elastic transversal strap (7), (both with and/or without tension).

It also incorporates two elastic straps (29,29'), (both with and/or without tension), to the external or internal central back part of the dynamic accessory for the protection and bracing of the tibial plateau, kneecap and knee (1), which cross at the level of the posterior cruciate ligament, and which extend longitudinally, towards the collateral ligaments, as well as, two elastic bands (28,28'), (both with and/or without tension), to the internal or external central front part of the dynamic accessory for the protection and bracing of the tibial plateau, kneecap and knee (1), which cross at the level of the anterior cruciate ligament, and extend longitudinally, towards the lateral ligaments.

The sides (17,18) of the sectioned pocket (2) have an alternating concave-convex shape with varying radius and angles on each side. The dynamic accessory for the protection and support of the tibial plateau, kneecap and knee (made in one continuous piece), embodied in this form, has 2 subsections of different length (25,26) ascending towards the thigh of the user. In the figure is presented details of the fastening strap (14) to the thigh of the user of the dynamic accessory for the protection and bracing of the tibial plateau, kneecap and knee (1) by the closing system of hooks and loops.

The central stabilizer of the tibial plateau (4) in a bowl-shape that reinforces the protection of the patellar tendon, as well as the anterior cruciate ligament. The lateral stabilizers of the tibial plateau and kneecap (10,11) located at the height of the external and internal of the tibial plateau and internal and external femoral condyles in a curved shape, with extensions in the form of a saw (15,16) protect the kneecap, with an encircling movement around the entire extension of the kneecap, that reinforces the protection of the external or lateral meniscus and of the internal or medial meniscus, as well as the correct location of the kneecap during movement. The two pairs of stabilizers surround the two lateral ligaments (13,13'), housed in the sectioned pockets (12, 12') are shown in the shape of flat interlocked chain links, and are used by surrounding the side above the superficial lateral of the kneecap, tibia, and femur, reinforcing the natural movement of the ligaments and their proper elasticity. The two subsections with different lengths (25,26) surround the kneecap on its anterior part and cross along the back of the knee on its upper edge, leaving the popliteal fossa free.

Figure 13 represents an alternative embodiment of the dynamic accessory for the protection and bracing of the tibial plateau, kneecap and knee (1) (made in one continuous piece, of elastic microfiber fabric, neoprene, foam padding, and breathable and non-breathable fabric), in the central section (20), that corresponds to the sectioned pocket (2) has a (9) strategically situated slit on one of its ends, through which the opposite end is introduced, which in this case (Figure 13) corresponds to the side section (22) of the dynamic accessory for the protection and support of the tibial plateau, kneecap and knee (1), so that it closes with a hook and loop system, allowing it to surround the perimeter of the users leg below the knee. In this embodied form, the two pairs of stabilizers surround the lateral ligaments (13,13') with the shape of a flat chain that is integrated in the pocket (2) and the configuration of the two sides (17,18) of the sectioned pocket (2) which present concave and convex curves, with different radius and angles on each of the sectioned side pockets.

## Claims

1. The dynamic accessory for the protection and bracing of the tibial plateau, kneecap and knee (1), (specially made from materials of elastic microfiber fabric, neoprene, foam padding, and breathable and non-breathable fabric), located perimetrically on the inferior part of the knee and held by hook and loop fasteners characterized because it is comprised of one continuous piece (1) contoured with curved and straight edges, encircling the inferior part of the knee, ascending in two subsections of different length (25, 26) through the contour of the kneecap and lateral areas of the knee, crossing above the superior part of the kneecap and wrapping around the thigh, while leaving the popliteal fossa free and with at least one sectioned pocket (2) providing a chamber of air, that houses the central stabilizers of the tibial plateau in a concave shape (4) and the lateral stabilizers of the tibial plateau and kneecap in a curving shape similar to the silhouette of a duck without a bill (10, 11) and its extensions in the shape of the edge of a saw (15,16), and the sectioned pockets (12, 12') that house the two pairs of stabilizers encircling the lateral ligaments in the form of a flat chain (13, 13'). The sides (17, 18) of the sectioned pocket (2) have the form of alternating concave-convex curves with different radius and angles on each side.

2. The dynamic accessory for the protection and bracing of the tibial plateau, kneecap and knee (1),(specially made from materials of elastic microfiber fabric, neoprene, foam padding, and breathable and non-breathable fabric), placed perimetrically on the inferior of the knee and held in place through a hook and loop closing system **characterized by** being made of a single continuous piece (1) contoured with curved and straight edges, wrapping around the inferior part of the knee while leaving the popliteal fossa free, with at least one sectioned pocket (2) providing a chamber of air and a space that houses the central stabilizer of the tibial plateau concave in shape (4) and to the lateral stabilizers of the tibial plateau and kneecap in a curved shape similar to a duck without a bill and with an oversized head (3, 5). The sides (17,18) of the sectioned pocket (2) have an alternating concave-convex shape with different radius and angles on each side.

3. The dynamic accessory for the protection and bracing of the tibial plateau, kneecap and knee (1), (specially made from materials of elastic microfiber fabric, neoprene, foam padding, and breathable and non-breathable fabric), placed perimetrically on the inferior of the knee and held in place through a hook and loop closing system and wrapping around the lateral ligaments according to Claim 1, characterized because the length of the stabilizers of the tibial plateau and kneecap in a curved shape similar to a silhouette of a duck without a bill (10, 11) with ends in the shape of the teeth of a saw (15, 16) is greater than the length of the stabilizers of the tibial plateau and kneecap with a curved shape similar to the silhouette of a duck without a bill and an oversized head (3, 5).

4. The dynamic accessory for the protection and bracing of the tibial plateau, kneecap and knee (1),(specially made from materials of elastic microfiber fabric, neoprene, foam padding, and breathable and non-breathable fabric), placed perimetrically on the low side of the knee and held in place through a hook and loop closing system and wrapping around the lateral ligaments according to Claim 1 characterized because of the length of the two pairs of stabilizers that encircle the lateral ligaments (13, 13') is greater than the length of the stabilizers of the tibial plateau and kneecap (10, 11), which end in the shape of the teeth of a saw (15, 16).

5. The dynamic accessory for the protection and bracing of the tibial plateau, kneecap and knee (1), (specially made from materials of elastic microfiber fabric, neoprene, foam padding, and breathable and non-breathable fabric), placed perimetrically on the low side of the knee and held in place through a hook and loop closing system and wrapping around the lateral ligaments according to Claim 1, characterized because in one of the preferred embodiments the configuration of the sectioned pocket (2) it is curved in shape at its superior ends (23, 24) and with a concave and convex curve in which each side has different radius and angles (17, 18).

6. The dynamic accessory for the protection and bracing of the tibial plateau, kneecap and knee (1), (specially made from materials of elastic microfiber fabric, neoprene, foam padding, and breathable and non-breathable fabric), placed perimetrically on the low part of the knee and held in place through a hook and loop closing system and wrapping around the lateral ligaments according to Claim 1, characterized because it incorporates an elastic longitudinal strap (6) (with and/or without tension) located on the middle part of its external side.

7. The dynamic accessory for the protection and bracing of the tibial plateau, kneecap and knee (1), (specially made from materials of elastic microfiber fabric, neoprene, foam padding, and breathable and non-breathable fabric), placed perimetrically on the lower side of the knee and held in place through a hook and loop closing system and wrapping around the lateral ligaments according to Claim 1, characterized because there is an elastic longitudinal strap (6) (with and/or without tension) located on the middle of the external face, complemented on its the posterior lateral external part of the dynamic accessory for the protection and bracing of the tibial plateau, kneecap, and knee (1) with an elastic transversal strap (7) (with and/or without tension).

8. The dynamic accessory for the protection and bracing of the tibial plateau, kneecap and knee (1),(specially made from materials of elastic microfiber fabric, neoprene, foam padding, and breathable and non-breathable fabric), placed perimetrically on the low side of the knee and held in place through a hook and loop closing system and wrapping around the lateral ligaments according to Claim 1, characterized because above the elastic longitudinal strap (6) (with and/or without tension), there is a bracing strap (27) superimposed with one or two buckles at one of its ends, and a hook and loop system to close it on the opposite end, through the posterior lateral external part of the dynamic accessory for the protection and bracing of the tibial plateau, kneecap and knee (1), under the elastic transversal strap (7) (with and/or without tension).

9. The dynamic accessory for the protection and bracing of the tibial plateau, kneecap and knee (1), (specially made from materials of elastic microfiber fabric, neoprene, foam padding, and breathable and non-breathable fabric), placed perimetrically on the low side of the knee and held in place through a hook and loop closing system and wrapping around the lateral ligaments according to Claim 1, characterized because in one of the preferred embodiments the bracing tongue (8), which is a single continuous piece attached on one of its ends (19), to the exterior face of the dynamic accessory for the protection and bracing of the tibial plateau, kneecap and knee (1), leaving the other end of the bracing tongue free (8). Once the dynamic accessory for the protection and bracing of the tibial plateau, kneecap and knee is both placed and braced (1), around the perimeter of the leg of the user at the inferior and lateral parts of the knee and kneecap, the bracing tongue (8) wraps the dynamic accessory for protection and bracing (1) and fixes itself at the level of the tibial area, through the hook and loop closing system provided by the bracing tongue (8) on its interior side, just as the dynamic accessory for protection and bracing (1) does on its exterior side.

10. The dynamic accessory for the protection and bracing of the tibial plateau, kneecap and knee (1), (specially made from materials of elastic microfiber fabric, neoprene, foam padding, and breathable and non-breathable fabric), placed perimetrically on the inferior of the knee and held in place through a hook and loop closing system and wrapping the lateral ligaments according to Claim 1, characterized because in one of its preferred embodiments the wrapping section through the inferior and frontal part of the knee is presented in three sections, a central part (20), which has a (9) strategically situated slit on one of its ends and two lateral sections (21, 22) one on each side of the central section (20).

11. The dynamic accessory for the protection and bracing of the tibial plateau, kneecap and knee (1), (specially made from materials based on elastic microfiber, neoprene, foams and breathable and non-breathable textiles), located perimetrically in the lower part of the knee by means of hook and loop closure elements for fabrics and wrapping around the lateral ligaments according to claim 1, characterized because it incorporates two elastic straps (28, 28 ') (with and/or without tension), joined together (by gluing, sewing, thermo -sealing or any fixing system), to the internal or external central front part of the dynamic complement of protection and support of the tibial plateau, patella and knee, which intersect at the level of the anterior cruciate ligament, and extend longitudinally, towards the lateral ligaments.

12. The dynamic accessory for the protection and bracing of the tibial plateau, kneecap and knee (1),(specially made from materials based on elastic microfiber, neoprene, foams and breathable and non-breathable textile), located perimetrically at the bottom of the knee by means of hook and loop closure elements and wrapping around the lateral ligaments according to claim 1, **characterized in that** it incorporates two elastic straps (29, 29') (with and/or without tension), joined together (by gluing, sewing, thermo-sealing or any fixation system), to the external or internal central back part of the dynamic complement of protection and support of the tibial plateau, patella and knee, which cross at the level of the posterior cruciate ligament, and extend longitudinally, towards the collateral ligaments .

13. The dynamic accessory for the protection and bracing of the tibial plateau, kneecap and knee (1), (specially made from materials of elastic microfiber fabric, neoprene, foam padding, and breathable and non-breathable fabric), placed perimetrically on the low part of the knee and held in place through a hook and loop closing system according to Claim 2, characterized because in one of its preferred embodiments, the configuration of the sectioned pocket (2) presents a concave-convex curved form on each of its ends (17, 18).

14. The dynamic accessory for the protection and bracing of the tibial plateau, kneecap and knee (1), (specially made from materials of elastic microfiber fabric, neoprene, foam padding, and breathable and non-breathable fabric), placed perimetrically on the inferior of the knee and held in place through a hook and loop closing system according to Claim 2, characterized because in the middle part of the exterior side there is an elastic longitudinal strap (6) (with and/or without tension).

15. The dynamic accessory for the protection and bracing of the tibial plateau, kneecap and knee (1), (specially made from materials of elastic microfiber fabric, neoprene, foam padding, and breathable and non-breathable fabric), placed perimetrically on the low part of the knee and held in place through a hook and loop closing system according to Claim 2, characterized because in the middle part of the exterior side there is an elastic longitudinal strap (6) (with and/or without tension), complemented by an elastic band (7) (with and/or without tension) on the posterior external side of the Dynamic accessory for the protection and bracing of the tibial plateau, kneecap, and knee (1).

16. The dynamic accessory for the protection and bracing of the tibial plateau, kneecap and knee (1), (specially made from materials of elastic microfiber fabric, neoprene, foam padding, and breathable and non-breathable fabric), placed perimetrically on the inferior of the knee and held in place through a hook and loop closing system according to Claim 2, characterized because above the elastic longitudinal strap (6) (with and/or without tension), there is a superimposed bracing strap (27), with one or two buckles at one of its ends, and the hook and loop closing system on the opposite end, through the external posterior lateral part of the Dynamic accessory for the protection and bracing of the tibial plateau, kneecap, and knee (1), under the elastic transversal strap (7) (with and/or without tension).

17. The dynamic accessory for the protection and bracing of the tibial plateau, kneecap and knee (1), (specially made from materials of elastic microfiber fabric, neoprene, foam padding, and breathable and non-breathable fabric), placed perimetrically below the knee and held in place through a hook and loop closing system according to Claim 2, characterized because one of its preferred embodiments presents a bracing tongue (8) attached by one of its ends (19) to the exterior face of the dynamic accessory for the protection and bracing (1) through the external hind lateral part of the dynamic accessory for the protection and bracing of the tibial plateau, kneecap and knee (1) leaving the other end of the bracing tongue free (8). Once the dynamic accessory for the protection and bracing of the tibial plateau, kneecap and knee (1) is both placed and braced, around the perimeter of the leg of the user at the inferior and lateral parts of the knee and kneecap, the bracing tongue (8) wraps around the dynamic accessory for protection and bracing (1) and fixes itself at the level of the tibial area, through the hook and loop closing system provided by the bracing tongue (8) on its interior side, just as the dynamic accessory for protection and bracing (1) does on its exterior side.

18. The dynamic accessory for the protection and support of the tibial plateau, kneecap and knee (1), (specially made from materials of elastic microfiber fabric, neoprene, foam padding, and breathable and non-breathable fabric), placed perimetrically below the knee and held in place through a hook and loop closing system according to Claim 2, characterized because one of the preferred embodiments has three sections, a central (20) that has a (9) strategically situated slit on one of its two ends and two lateral sections (21,22) on either side of the central section (20).

19. The dynamic accessory for the protection and support of the tibial plateau, kneecap and knee (1),(specially made from materials of elastic microfiber, neoprene, foams and breathable and non-breathable textile), located perimetrically in the lower part of the knee by means of hook and loop closure elements for materials and enveloping the lateral ligaments according to claim 2, **characterized in that** the dynamic complement closes on itself, with the joint connection between its two lateral ends (30, 30'), (by gluing, sewing, heat-sealing or any fixing system), surrounding the perimeter below the knee, and in an enveloping maneuver over the kneecap and knee, without the need to use hook and loop closure systems.

20. The dynamic accessory for the protection and support of the tibial plateau, kneecap and knee (1),(specially constituted from materials based on elastic microfiber fabrics, neoprene, foams and breathable and non-breathable textiles), located to cover the perimeter around the lower part of the knee by means of hook and loop closure elements for fabrics and wrapping in the lateral ligaments according to claim 2, **characterized in that** it increases the height of its front central section, which corresponds to the sectioned pocket (2) and its sides (17, 18), to cover a greater surface area of the patella, and increases the height of posterior lateral part of the dynamic accessory for the protection and support of the tibial plateau, kneecap and knee (1), so that in an enveloping maneuver, it closes on a greater surface area of the patella, aiding in the knee stability.

21. The dynamic accessory for the protection and support of the tibial plateau, kneecap and knee (1), (specially made from materials based on elastic microfiber fabrics, neoprene, foams and breathable and non-breathable textiles), located perimetrically around the lower part of the knee by means of hook and loop closure elements for fabrics and wrapping around the lateral ligaments according to claim 2, **characterized in that** it incorporates two elastic tapes (28, 28') (with and/or without tension), joined together (by gluing, sewing, heat-sealing or any fixing system), to the internal or external central front part of the complement to the dynamic protection and support of the tibial plateau, patella and knee, which intersect at the level of the anterior cruciate ligament, and extend longitudinally, towards the lateral ligaments.

22. The dynamic accessory for the protection and bracing of the tibial plateau, kneecap and knee (1), (specially made from materials of elastic microfiber fabrics, neoprene, foams and breathable and non-breathable textile), located perimetrically around the lower part of the knee by means of hook and loop closure elements for fabrics and wrapping in the lateral ligaments according to claim 2, **characterized in that** it incorporates two elastic tapes (29, 29') (with and/or without tension), joined together (by gluing, sewing, thermo-sealing or any fixing system), to the external or internal central back part of the complement dynamic protection and support of the tibial plateau, patella and knee, which cross at the level of the posterior cruciate ligament, and extend longitudinally, towards the collateral ligaments.

23. The dynamic accessory for the protection and bracing of the tibial plateau, kneecap and knee (1), (specially made from materials based on elastic microfiber fabrics, neoprene, foams and breathable and non-breathable textiles), located perimetrically around the lower part of the knee by means of hook and loop closure elements for fabrics and wrapping in the lateral ligaments according to claim 2, **characterized by** the inclusion of two stabilizers in the shape of an elongated oval (31, 31') housed in the sectorized pocket (2), located in line with the external and internal lateral area of the knee.

24. The dynamic accessory for the protection and support of the tibial plateau, kneecap and knee (1),(specially made from materials of elastic microfiber, neoprene, foams and breathable and non-breathable textile), located perimetrically in the lower part of the knee by means of hook and loop closure elements for materials and enveloping the lateral ligaments according to claim 2, **characterized in that** the dynamic complement closes on itself, with the joint connection between its two lateral ends (30, 30'), (by gluing, sewing, heat-sealing or any fixing system), surrounding the perimeter below the knee, and in an enveloping maneuver over the kneecap and knee, without the need to use hook and loop closure systems.
